# EUROPEAN PATENT APPLICATION

(11) **EP 4 335 412 A1**
(43) Date of publication of application: **13.03.2024**
(21) Application number: 23194200.4
(22) Date of filing: 30.08.2023
(51) Int. Cl.: A61F 2/24

(54) **PROSTHETIC HEART VALVE WITH NON-ABRASIVE OUTFLOW REGION**

(30) Priority: 12.09.2022 US 202263405729 P; 21.08.2023 US 202318452794
(71) Applicant: Medtronic Vascular Inc., Santa Rosa, CA 95403 (US)
(72) Inventor: Peterson, Justin R., Santa Rosa, 95403 (US); Kari, Stuart E., Santa Rosa, 95403 (US)
(74) Representative: Zimmermann & Partner Patentanwälte mbB

(57) **Abstract**

The techniques of this disclosure generally relate to a valve prosthesis including a one piece molded prosthetic valve and a stent structure having an outflow crown ring. The outflow crown ring includes inferior crowns, superior crowns, and outflow crown struts connected to the superior crowns and the inferior crowns. An internal radius of the superior crowns is within the range of 0.3 to 0.5 millimeters (mm). The internal radius of the superior crowns is made sufficiently large to prevent flaring outward of the superior crowns on deployment and to make the superior crowns atraumatic. This reduces and essentially eliminates the chance of contact with the aortic sinus or the ascending aorta. Further, in the event there is contact, the atraumatic shape of the superior crowns minimizes the abrasion of the vessel.

## Description

### CROSS-REFERENCE TO RELATED APPLICATIONS

The present application claims priority to U.S. Provisional Application No. 63/405,729, filed September 12, 2022, the contents of which are incorporated by reference herein in their entirety.

### FIELD

The present technology is generally related to transcatheter heart valves.

### BACKGROUND

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

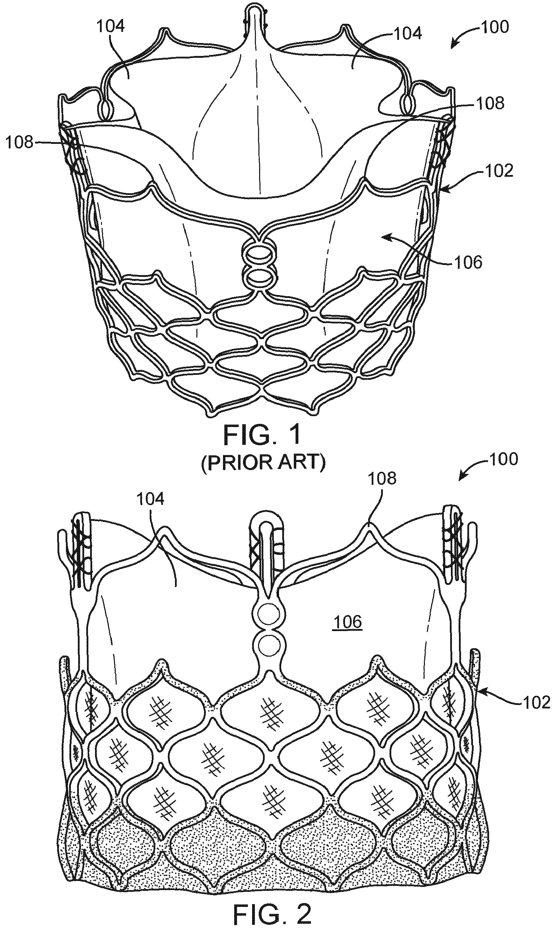

Recently, flexible prosthetic valves supported by stent structures that can be delivered percutaneously using a catheter-based delivery system have been developed for heart and venous valve replacement. FIG. 1 is a perspective view of a valve prosthesis 100 in accordance with the prior art. FIG. 2 is a side view of valve prosthesis 100 of FIG. 1 in accordance with the prior art. Referring to FIGS. 1 and 2, valve prosthesis 100 may include a balloon-expandable stent structure 102 with valve leaflets 104 attached to the interior of stent structure 102. Valve leaflets 104 may be parts of a 3D single piece prosthetic valve 106.

Valve prosthesis 100 can be reduced in diameter, by crimping onto a balloon catheter, and advanced through the venous or arterial vasculature. Once valve prosthesis 100 is positioned at the treatment site, for instance within an incompetent native valve, stent structure 102 may be expanded to hold valve prosthesis 100 firmly in place.

When designing a valve prosthesis such as valve prosthesis 100, valve-frame integration and frame mechanical performance often have competing needs or requirements. For example, when attaching the valve to the frame during valve-frame integration, the valve itself needs to be reinforced to the frame at certain locations without hindering mechanical performance of the frame. Embodiments hereof relate to an improved balloon-expandable transcatheter valve prosthesis configured to minimize tradeoffs between the above-described competing needs.

### SUMMARY

The techniques of this disclosure generally relate to a valve prosthesis including a one piece molded prosthetic valve and a stent structure having an outflow crown ring. The outflow crown ring includes inferior crowns, superior crowns, and outflow crown struts connected to the superior crowns and the inferior crowns. An internal radius of the superior crowns is within the range of 0.3 to 0.5 millimeters (mm). The internal radius of the superior crowns is made sufficiently large to prevent flaring outward of the superior crowns on deployment and to make the superior crowns atraumatic. This reduces the chance of contact with the aortic sinus or the ascending aorta. Further, in the event there is contact, the atraumatic shape of the superior crowns minimizes the abrasion of the vessel.

In one aspect, the present disclosure provides a valve prosthesis including a one piece molded prosthetic valve and a stent structure having an outflow portion. The outflow portion is tapered and has a minimum diameter at an outflow end of the valve prosthesis. The valve prosthesis is a supra-annular valve and has a tapered shape to reduce the chance of contact with the aortic sinus or the ascending aorta and the associated possible vessel injury.

In another aspect, the present disclosure provides a valve prosthesis including a one piece molded prosthetic valve having valve leaflets including free edges. The valve prosthesis further includes a stent structure having an outflow portion including an outflow crown ring. The free edges of the valve leaflets are inferior to the outflow crown ring. By avoiding overlap of and contact between the valve leaflets and the outflow crown ring, damage of the free edges of the valve leaflets from the outflow crown ring is avoided, e.g., during (transaortic heart valve) THV crimp and deployment.

Further disclosed herein is a valve prosthesis including a one piece molded prosthetic valve and a stent structure having an outflow crown ring, wherein the outflow crown ring includes inferior crowns, superior crowns, and outflow crown struts connected to the superior crowns and the inferior crowns, wherein an internal radius of the superior crowns is within the range of 0.3 to 0.5 millimeters (mm), wherein the internal radius of the superior crowns is made sufficiently large to prevent flaring outward of the superior crowns on deployment and to make the superior crowns atraumatic. This reduces and essentially eliminates the chance of contact with the aortic sinus or the ascending aorta, and further, in the event there is contact, the atraumatic shape of the superior crowns minimizes the abrasion of the vessel.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a valve prosthesis in accordance with the prior art.
FIG. 2 is a side view of the valve prosthesis of FIG. 1 in accordance with the prior art.
FIG. 3 is a side view of a valve prosthesis in an expanded configuration in accordance with one embodiment.
FIG. 4 is an outflow end view of a prosthetic valve of the valve prosthesis of FIG. 3 in accordance with one embodiment.
FIG. 5 is a perspective view of the prosthetic valve of the valve prosthesis of FIG. 3 in accordance with one embodiment.
FIG. 6 is a side view of a valve prosthesis in an expanded configuration in accordance with another embodiment.
FIG. 7 is a side view of an expanded tapered balloon used to deploy the valve prosthesis of FIG. 6 with a tapered shape in accordance with one embodiment.
FIG. 8 is a side view of a valve prosthesis in an expanded configuration in accordance with another embodiment.
FIG. 9 is a side view of an expanded partially tapered balloon used to deploy the valve prosthesis of FIG. 8 with a tapered shape outflow portion in accordance with one embodiment.
FIG. 10 is a side view of struts in a pre-expansion configuration of a stent structure similar to a stent structure of the valve prosthesis of FIG. 3 in accordance with one embodiment.
FIG. 11 is a side view of the struts of FIG. 10 in an expanded configuration in accordance with one embodiment.
FIG. 12 is a side view of a valve prosthesis in an expanded configuration in accordance with another embodiment.
FIG. 13 is an enlarged view of a region XIII of the valve prosthesis of FIG. 12 in accordance with one embodiment.
FIG. 14 is a side view of a valve prosthesis in an expanded configuration in accordance with another embodiment.

### DETAILED DESCRIPTION

Specific embodiments are now described with reference to the figures, wherein like reference numbers indicate identical or functionally similar elements. The terms "distal" and "proximal", when used in the following description to refer to a native vessel, native valve, or a device to be implanted into a native vessel or native valve, such as a heart valve prosthesis, are with reference to the direction of blood flow. Thus, "distal" and "distally", also referred to as the "outflow" or "outflow direction", respectively, refer to positions in a downstream direction with respect to the direction of blood flow. The terms "proximal" and "proximally", also referred to as the "inflow" or "inflow direction", respectively, refer to positions in an upstream direction with respect to the direction of blood flow. Proximal is also sometimes referred to as inferior and distal is sometimes referred to as superior.

Although the description is in the context of treatment of an aortic heart valve, embodiments may also be used where it is deemed useful in other valved intraluminal sites that are not in the heart. For example, embodiments may be applied to other heart valves or venous valves as well.

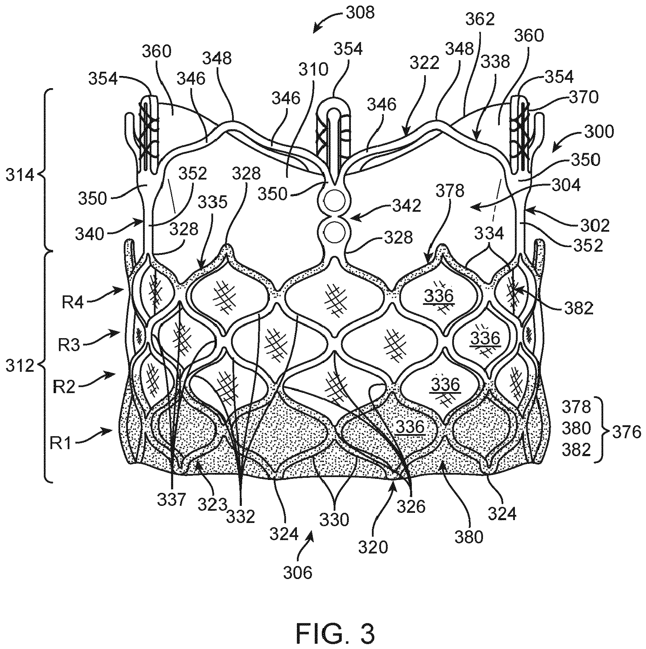

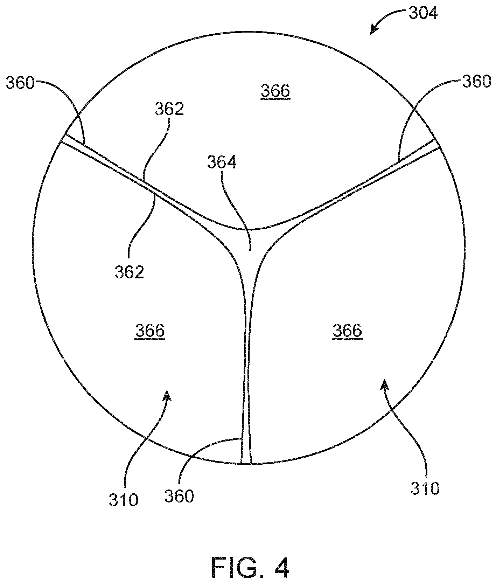

FIG. 3 is a side view of a transcatheter valve prosthesis 300 in the expanded configuration in accordance with one embodiment. FIG. 4 is an outflow end view of a prosthetic valve 304 of valve prosthesis 300 of FIG. 3 in accordance with one embodiment. Valve prosthesis 300 has a radially-expandable stent structure 302, sometimes called the frame, and prosthetic valve 304. Stent structure 302 is generally tubular, and is mechanically or balloon expandable, having a crimped configuration for delivery within a vasculature and an expanded configuration for deployment within a native heart valve. When valve prosthesis 300 is deployed within the valve annulus of a native heart valve, stent structure 302 of valve prosthesis 300 is configured to be radially expanded within native valve leaflets of the patient's defective valve, to thereby retain the native valve leaflets in a permanently open state. In embodiments hereof, valve prosthesis 300 is configured for replacement of an aortic valve such that an inflow end 306, i.e., the proximal end, of valve prosthesis 300 extends into and anchors within the aortic annulus of a patient's left ventricle, while an outflow end 308, i.e., the distal end, of valve prosthesis 300 is positioned within the aortic sinuses.

Stent structure 302 of valve prosthesis 300 may be a unitary frame or scaffold that supports prosthetic valve 304 including one or more valve leaflets 310 within the interior of stent structure 302. Prosthetic valve 304 is capable of blocking flow in one direction to regulate flow there-through via valve leaflets 310 that may form a bicuspid or tricuspid replacement valve. FIG. 4 illustrates that prosthetic valve 304 has three valve leaflets 310, i.e., prosthetic valve 304 has a tricuspid leaflet configuration, although a bicuspid leaflet configuration may be used in other embodiments. More particularly, as valve prosthesis 300 is configured for placement within a native aortic valve which typically has three leaflets, prosthetic valve 304 may include three valve leaflets 310. However, valve prosthesis 300 is not required to have the same number of leaflets as the native valve. If valve prosthesis 300 is alternatively configured for placement within a native valve having two leaflets such as the mitral valve, prosthetic valve 304 may include two or three valve leaflets 310.

Stent structure 302 is balloon-expandable. As such, stent structure 302 is made from a plastically deformable material such that when expanded by a dilatation balloon, stent structure 302 maintains its radially expanded configuration. Stent structure 302 may be formed from stainless steel such as 316L or other suitable metal, such as platinum iridium, cobalt chromium alloys such as MP35N or L605, or various types of polymers or other similar materials, including the materials coated with various surface deposits to improve clinical functionality. Stent structure 302 is configured to be rigid such that it does not deflect or move when subjected to in-vivo forces, or such that deflection or movement is minimized when subjected to in-vivo forces.

Stent structure 302 includes an inflow portion 312 and an outflow portion 314. Stent structure 302 is a tubular component defining a central lumen or passageway, and has an inflow end 320 and an outflow end 322. When expanded, a diameter of inflow end 320 of stent structure 302 is substantially the same as a diameter of outflow end 322 of stent structure 302 in one embodiment.

Inflow portion 312 extends distally from inflow end 320 of stent structure 302. Inflow portion 312 includes inflow crowns 324, central crowns 326, outflow crowns 328, inflow struts 330, central struts 332, and outflow struts 334. Inflow portion 312 further includes an inflow crown ring 323 defined by inflow crowns 324, inflow struts 330, and central crowns 326. Inflow portion further includes an outflow crown ring 335 defined by outflow crowns 328, outflow struts 334, and central crowns 326. Inflow portion 312 generally extends between inflow crown ring 323 and outflow crown ring 335.

In between inflow end 320 and outflow end 322 of stent structure 302, inflow portion cells 336, sometimes called side openings, are formed in rows, and more particularly, in four rows R1, R2, R3, and R4. Each row R1, R2, R3, R4 includes 12 inflow portion cells 336. Inflow portion cells 336 of the proximal most row R1 are defined by inflow crowns 324, inflow struts 330, central crowns 326, and central struts 332. Inflow portion cells 336 of the next most proximal rows R2, R3 are defined by central crowns 326 and central struts 332. Inflow portion cells 336 of the distal most row R4 are defined by outflow crowns 328, outflow struts 334, central crowns 326, and central struts 332. Generally, inflow portion cells 336 are diamond-shaped openings having the same or identical shaped, sometimes are called symmetric.

Generally, a crown is defined where two struts connect and a node is defined as a region where two crowns connect. Accordingly, inflow crowns 324 are defined where inflow struts 330 connect. Outflow crowns 328 are defined where outflow struts 334 connect. Central crowns 326 are defined where inflow struts 330 connect, where outflow struts 334 connect, and where central struts 332 connect. Central nodes 337 are defined where central crowns 326 connect.

Outflow portion 314 is formed proximate to outflow end 322 of stent structure 102 and between outflow end 322 and inflow portion 312. Outflow portion 314 includes an outflow crown ring 338, commissure posts 340, and non-commissure posts 342. Outflow portion 314 can be configured in a shape that forms a central lumen or passageway.

Outflow crown ring 338 includes outflow crown struts 346, superior crowns 348, and inferior crowns 350. Each outflow crown struts 346 is connected on one end to an adjacent outflow crown strut 346 at a superior crown 348 and on the opposite end to an adjacent outflow crown strut 346 at an inferior crown 350. Inferior crowns 350 are connected to either a commissure post 340 or a non-commissure post 342. More particularly, every other inferior crown 350 is connected to a commissure post 340 and every other inferior crown 350 is connected to a non-commissure post 342 in an alternating repeating arrangement.

Non-commissure posts 342, sometimes called axial frame members 342, extend longitudinally between and connect inferior crowns 350 of outflow crown ring 338 and outflow crowns 328 of inflow portion 312. In accordance with this embodiment, non-commissure posts 342 are shaped as a figure eight and are used as markers for depth of implant as well as clocking of valve prosthesis 300. As used herein, longitudinally is in a direction parallel with the longitudinal axis, radially is perpendicular and in a radial direction from the longitudinal axis, and circumferentially is in a plane perpendicular to the longitudinal axis and in a direction along the circumference of valve prosthesis 300.

Commissure posts 340 extend longitudinally and include axial frame members 352 and trident posts 354. Axial frame members 352 extend longitudinally between and connect inferior crowns 350 of outflow crown ring 338 and outflow crowns 328 of inflow portion 312. Trident posts 354 extend in a cantilever fashion and in the outflow or distal direction from inferior crowns 350 of outflow crown ring 338. Axial frame members 352 and trident posts 354 are parallel with one another and are segments of commissure posts 340, which are linear members.

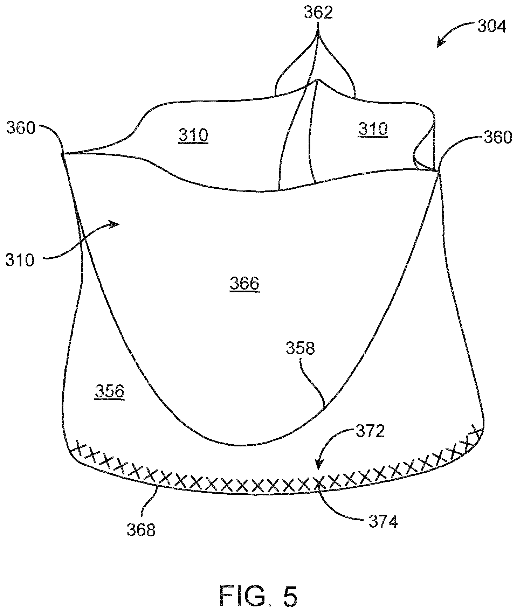

FIG. 5 is a perspective view of prosthetic valve 304 of valve prosthesis 300 of FIG. 3 in accordance with one embodiment. FIGS. 3, 5 illustrate prosthetic valve 304 in an open state whereas FIG. 4 illustrates prosthetic valve 304 in a closed state.

Referring now to FIGS. 3-5 together, prosthetic valve 304 is a one piece three dimensional (3D) molded structure in accordance with this embodiment. Illustratively, a single cylindrical piece is molded to form prosthetic valve 304. Accordingly, although various structures of prosthetic valve 304 are discussed below, prosthetic valve 304 is integral, i.e., formed from a single piece and not a plurality of separate pieces connected together.

Prosthetic valve 304 may be made of pericardial material; however, may instead be made of another material. Natural tissue for prosthetic valve 304 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as prosthetic valve 304 include DACRON polyester commercially, other cloth materials, nylon blends, polymeric materials, and vacuum deposition nitinol fabricated materials. One polymeric material from which prosthetic valve 304 can be made is an ultra-high molecular weight polyethylene material. With certain materials, it may be desirable to coat one or both sides of prosthetic valve 304 with a material that will prevent or minimize overgrowth. It is further desirable that the material is durable and not subject to stretching, deforming, or fatigue.

Prosthetic valve 304 includes valve leaflets 310 and a valve inflow cylinder 356 proximal of valve leaflets 310. For example, valve inflow cylinder 356 is the remaining unmolded portion of the cylindrical material used to form prosthetic valve 304 and valve leaflets 310 are the molded portion.

Valve leaflets 310 are defined by cusps 358, commissures 360, and free edges 362. Adjoining pairs of valve leaflets 310 are attached to one another at their lateral ends to form commissures 360, with free edges 362 of valve leaflets 310 forming coaptation edges that meet in an area of coaptation 364. The region within cusps 358, commissures 360, and free edges 362 are sometimes referred to as a belly 366 of valve leaflets 310.

Valve inflow cylinder 356 has a cylindrical inflow end 368, sometimes called a nadir 368. Valve inflow cylinder 356 extends in the outflow direction from inflow end 368 as a cylinder to cusps 358, which form the outflow end of valve inflow cylinder 356.

Prosthetic valve 304 is disposed within and secured to at least trident posts 354 of commissure posts 340 of stent structure 302. In addition, prosthetic valve 304 may also be disposed within and secured to inflow portion 312 of stent structure 302. More particularly, commissures 360 are attached to trident posts 354, e.g., with commissure stitching 370. Further, a margin of attachment (MOA) 372 of valve inflow cylinder 356, e.g., a region directly adjacent inflow end 368, is attached to inflow portion 312, e.g., with MOA stitching 374. Although a particular location for MOA 372 adjacent inflow end 368 is illustrated in FIG. 5 and discussed above, in other embodiments, MOA 372 is in a different location. For example, MOA 372 is a parabolic MOA adjacent cusps 358. Other locations for MOA 372 are possible in yet other embodiments.

Valve prosthesis 300 further includes a skirt 376, e.g., formed of graft material, which encloses or lines a portion of stent structure 302. Margin of attachment (MOA) 372 of valve inflow cylinder 356 is sutured or otherwise securely and sealingly attached to the interior surface of skirt 376 and to inflow portion 312, e.g., with MOA stitching 374.

Skirt 376 may enclose or line stent structure 302. Skirt 376 may be a natural or biological material such as pericardium or another membranous tissue such as intestinal submucosa. Alternatively, skirt 376 may be a low-porosity woven fabric, such as polyester, Dacron fabric, or PTFE, which creates a one-way fluid passage. In one embodiment, skirt 376 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side.

In accordance with this embodiment, skirt 376 includes an outflow end outer skirt 378, and inflow end outer skirt 380, and an inner skirt 382. Outflow end outer skirt 378 is located on the outer surface of outflow crowns 328 and outflow struts 334 of inflow portion 312. Inflow end outer skirt 380 is located on the outer surface of inflow crowns 324, inflow struts 330, central crowns 326, and central struts 332. More particularly, inflow end outer skirt 380 covers first row R1 of inflow portion cells 336. Inner skirt 382 is located on the inner surface of inflow portion 312 and extends between inflow crown ring 323 and outflow crown ring 335.

Delivery of valve prosthesis 300 may be accomplished via a percutaneous transfemoral approach or a transapical approach directly through the apex of the heart via a thoracotomy, or may be positioned within the desired area of the heart via different delivery methods known in the art for accessing heart valves. During delivery, valve prosthesis 300 remains compressed until it reaches a target diseased native heart valve, at which time a balloon of a delivery system is inflated in order to radially expand valve prosthesis 300 in situ. Valve prosthesis 300 is configured to be expanded within the native valve leaflets of the patient's defective valve, to thereby retain the native valve leaflets in a permanently open state. The delivery system is then removed and transcatheter valve prosthesis 300 remains deployed within the native target heart valve.

In accordance with this embodiment, superior crowns 348 of outflow crown ring 338 are made to reduce or eliminate the chance of contact with the aortic sinus or the ascending aorta and the associated possible vessel injury. More particularly, the internal radius of superior crowns 348 is made sufficiently large to prevent flaring outward of superior crowns 348 on deployment and to make superior crowns 348 atraumatic. In one embodiment, the internal radius of superior crowns 348 is within the range of 0.3 to 0.5 millimeters (mm). Superior crowns 348 are sometime called the outflow crowns 348.

In this manner, superior crowns 348 remain within (lie within) the cylindrical shape of stent structure 302 instead of radially flaring outward, or the outward flaring is negligible. This reduces the chance of contact with the aortic sinus or the ascending aorta. Further, in the event there is contact, the blunt or atraumatic shape of superior crowns 348 minimizes the erosion of the vessel wall.

In contrast, referring to FIGS. 1-2, outflow crowns 108 of stent structure 102 of valve prosthesis 100 have a small internal radius forming a traumatic shape. It is believed that the internal radius of outflow crowns 108 is on the order of 0.1 mm. Accordingly, outflow crowns 108 pose a vessel trauma/injury issue if they make repetitive contact with the aortic sinus or ascending aorta. In addition, the small internal radius of outflow crowns 108 makes outflow crowns 108 flare radially on deployment further increasing the chance of contact with the aortic sinus or ascending aorta.

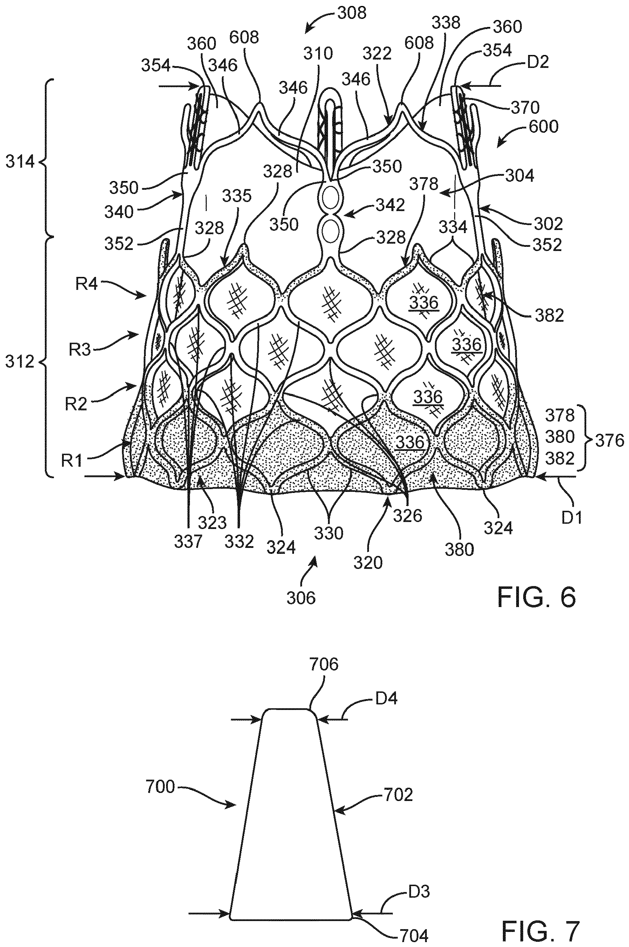

FIG. 6 is a side view of a transcatheter valve prosthesis 600 in the expanded configuration in accordance with another embodiment. Valve prosthesis 600 of FIG. 6 is similar to valve prosthesis 300 of FIG. 3 and only the significant differences are discussed below.

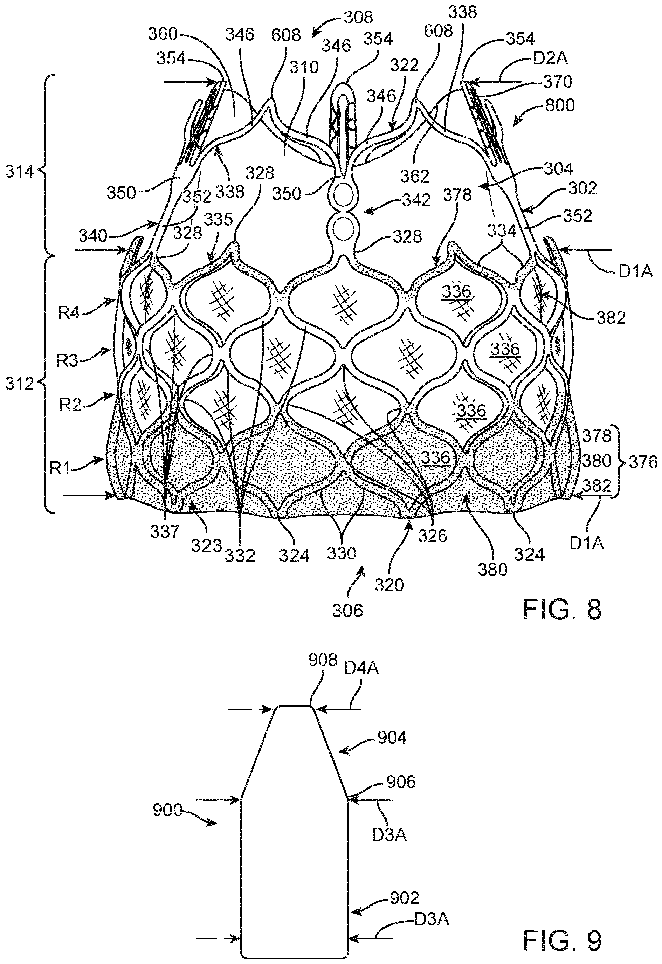

In accordance with this embodiment, valve prosthesis 600 is a supra-annular valve. Valve prosthesis 600 has a tapered shape to reduce the diameter of outflow portion 314, sometimes called the outflow region, and reduce the chance of contact with the aortic sinus or the ascending aorta and the associated possible vessel injury. More particularly, valve prosthesis 600 has the shape of a cone frustum, i.e., the shape obtained by cutting a cone from the top, also called a truncated cone. Valve prosthesis 600 has a first diameter D 1 at inflow end 306 and second diameter D2 at outflow end 308, second diameter D2 being smaller than first diameter D1. Valve prosthesis 600 uniformly tapers between first diameter D1 at inflow end 306 and second diameter D2 at outflow end 308 in accordance with this embodiment. In other embodiments, valve prosthesis 600 may have uniform diameter segments and/or segments that have varying tapers, e.g., such an example is discussed in reference to FIG. 8 below.

As valve prosthesis 600 has a tapered shape and thus minimal change of contact with the vessel, valve prosthesis 600 is illustrated having superior crowns 608 similar to outflow crowns 108 of valve prosthesis 100 of FIGS. 1-2. However, in another embodiment, valve prosthesis 600 has atraumatic superior crowns similar to superior crowns 348 of valve prosthesis 300 of FIG. 3.

FIG. 7 is a side view of an expanded tapered balloon 700 used to deploy valve prosthesis 600 of FIG. 6 with a tapered shape in accordance with one embodiment. Referring to FIGS. 6 and 7 together, to provide valve prosthesis 600 with a tapered shape, valve prosthesis 600 is deployed using tapered balloon 700. Tapered balloon 700 has a tapered outer surface 702 having a greater third diameter D3 at a distal end 704 than a fourth diameter D4 at a proximal end 706. The distal end of a delivery system including tapered balloon 700 is usually identified to the end that is farthest from the operator/handle while the proximal end is the end nearest the operator/handle.

As tapered balloon 700 is inflated to expand valve prosthesis 600 to the form illustrated in FIG. 6, the tapered shape of tapered balloon 700 causes stent structure 302, and thus valve prosthesis 600, to assume the tapered shape.

FIG. 8 is a side view of a transcatheter valve prosthesis 800 in the expanded configuration in accordance with another embodiment. Valve prosthesis 800 of FIG. 8 is similar to valve prosthesis 600 of FIG. 6 and only the significant differences are discussed below.

In accordance with this embodiment, valve prosthesis 800 is a supra-annular valve. Valve prosthesis 800 has tapered outflow portion 314 to reduce the chance of contact with the aortic sinus or the ascending aorta and the associated possible vessel injury. More particularly, outflow portion 314 has the shape of a cone frustum, i.e., the shape obtained by cutting a cone from the top, also called a truncated cone. Inflow portion 312 is in the shape of a uniform diameter cylinder.

More particularly, valve prosthesis 800 has a first diameter D1A at inflow portion 312. Outflow portion 314 has first diameter D1A where outflow portion 314 connects to inflow portion 312 and a second diameter D2A at outflow end 308, second diameter D2A being smaller than first diameter D1A. In accordance with this embodiment, second diameter DA2 is the minimum diameter of outflow portion 314 and generally of valve prosthesis 800. Outflow portion 314 uniformly tapers between first diameter D1A and second diameter D2A in accordance with this embodiment.

FIG. 9 is a side view of an expanded partially tapered balloon 900 used to deploy valve prosthesis 800 of FIG. 8 with a tapered shape outflow portion 314 in accordance with one embodiment. Referring to FIGS. 8 and 9 together, to provide inflow portion 312 with a cylindrical shape and outflow portion 314 with a tapered shape, valve prosthesis 800 is deployed using partially tapered balloon 900. Partially tapered balloon 900 has a cylindrical distal portion 902 and a proximal tapered portion 904. Cylindrical distal portion 902 has a uniform third diameter D3A. Proximal tapered portion 904 tapers at its distal end 906 from diameter D3A at cylindrical distal portion 902 to a smaller diameter D4A at a proximal end 908. As partially when tapered balloon 900 is inflated to expand valve prosthesis 800 to the form illustrated in FIG. 8, the partially tapered shape of partially tapered balloon 900 causes stent structure 302, and thus valve prosthesis 800, to assume the partially tapered shape.

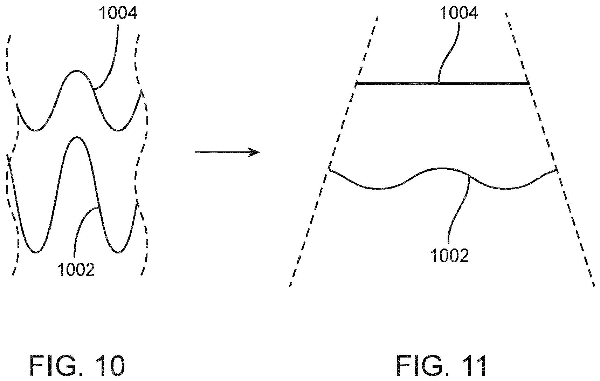

Although valve prosthesis 600, 800 are discussed above as being expanded by balloons 700, 900 to assume their shapes, in other embodiments, stent structure 302 is designed to non-uniformly expand to provide the shapes using a cylindrical balloon. In one embodiment, strut lengths are tailored along the height of stent structure 302 to create a tapered expansion response as discussed below in reference to FIGS. 10 and 11.

FIG. 10 is a side view of struts 1002, 1004 in a pre-expansion configuration of a stent structure similar to stent structure 302 of valve prosthesis 300 of FIG. 3 in accordance with one embodiment. FIG. 11 is a side view of struts 1002, 1004 of FIG. 10 in an expanded configuration in accordance with one embodiment.

Referring now to FIGS. 10 and 11 together, struts 1002 are longer than struts 1004. Accordingly, during expansion of the stent structure including struts 1002, 1004 from the pre-expansion state illustrated in FIG. 10 to the expanded state illustrated in FIG. 11, struts 1004 will reach their circumferential limits of expansion before struts 1002. Accordingly, struts 1004 will prevent further expansion of the associated portion of the stent structure before struts 1002 thus providing a tapered expansion response.

Although two sets of struts 1002, 1004 are set forth, in light of this disclosure, those of skill in the art will understand that two or more struts of different lengths can be provided to give a desired expansion response, for example, to assume the shapes as illustrated in FIGS. 6 and 8. Struts 1002, 1004 are representative of one or more of struts 330, 332, 334, 346.

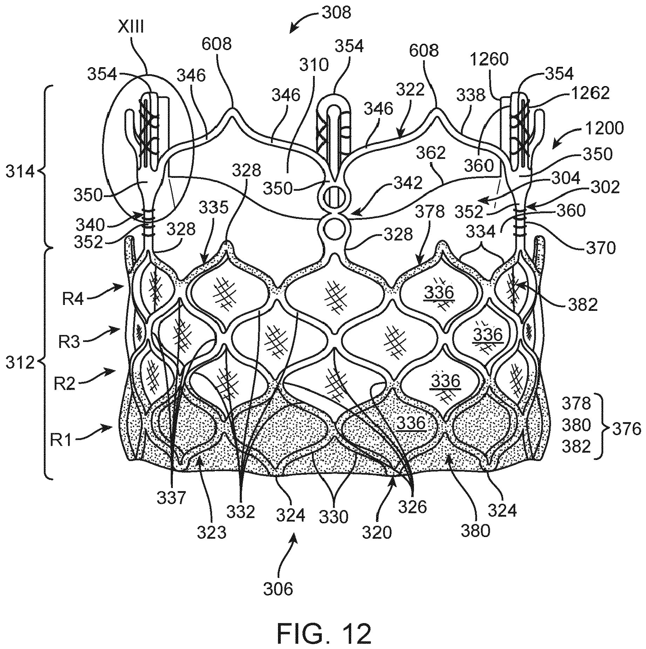

FIG. 12 is a side view of a transcatheter valve prosthesis 1200 in the expanded configuration in accordance with another embodiment. Valve prosthesis 1200 of FIG. 12 is similar to valve prosthesis 600 of FIG. 6 and only the significant differences are discussed below.

Referring now to FIG. 12, in accordance with this embodiment, free edges 362 of valve leaflets 310 are located in the inflow direction, sometimes called the proximal or inferior direction, from outflow crown ring 338. Generally, valve leaflets 310 are located in the inflow direction from outflow crown ring 338 including inferior crowns 350 and thus there is no overlap of valve leaflets 310 and outflow crown ring 338. More particularly, there is a longitudinal space between free edges 362 of valve leaflets 310 and outflow crown ring 338 insuring there is no contact between valve leaflets 310 and outflow crown ring 338.

By avoiding overlap of and contact between valve leaflets 310 and outflow crown ring 338, as outflow crown ring 338 opens during deployment, damage, e.g., shearing, of free edges 362 of valve leaflets 310 from outflow crown ring 338 is avoided. Further, pinning of valve leaflets 310 between the expanding balloon and outflow crown ring 338 during deployment is avoided. Pinning can prevent valve leaflets 310 from unfolding and causes stresses at free edges 362 and commissures 360 which causes "peeling", "splits", "cuts", "sheering", "tensile failure", and/or other damage to valve leaflets 310.

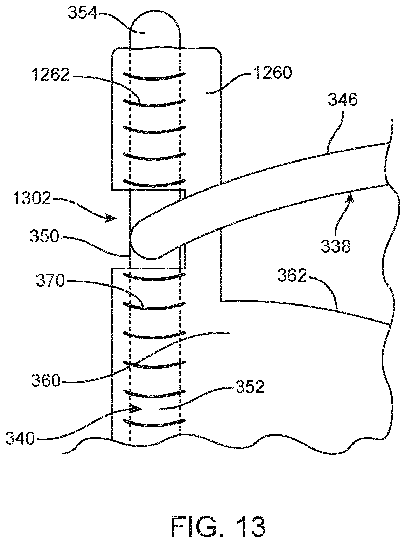

FIG. 13 is an enlarged view of a region XIII of valve prosthesis 1200 of FIG. 12 in accordance with one embodiment. Referring now to FIGS. 12 and 13 together, in accordance with this embodiment, valve prosthesis 300 includes protruding commissures 1260 extending in the outflow direction from commissures 360. Protruding commissures 1260 are post or flap shaped members that are attached to trident posts 354 with trident stitching 1262. Protruding commissures 1260 extend in the outflow direct from commissures 360 to overlap and be superior to inferior crowns 350 where outflow crown struts 346 connect to commissure posts 340. In other words, protruding commissures 1260 are attached to trident posts 354 superior to takeoffs of outflow crown struts 346. In one embodiment, protruding commissures 1260 include notches 1302 through which outflow crown struts 346 extend from commissure posts 340.

In accordance with this embodiment, commissures 360 are attached to axial frame members 352 of commissure posts 340 with commissure stitching 370. Commissures 360 are attached to axial frame members 352 inferior to inferior crowns 350 where outflow crown struts 346 connect to commissure posts 340.

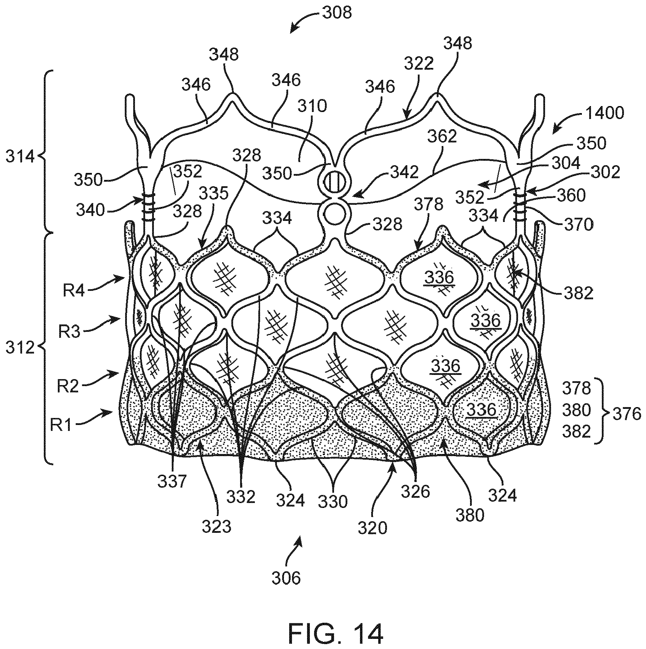

Although one attachment of protruding commissures 1260 and commissures 360 to commissure posts 340 is illustrated and discussed above to located free edges 362 inferior (below) outflow crown ring 338, in other embodiments, other attachment mechanisms of prosthetic valve 304 to stent structure 302 to located free edges 362 inferior (below) outflow crown ring 338 are used in other embodiments. One such example is discussed below in reference to FIG. 14.

FIG. 14 is a side view of a transcatheter valve prosthesis 1400 in the expanded configuration in accordance with another embodiment. Valve prosthesis 1400 of FIG. 14 is similar to valve prosthesis 1200 of FIG. 12 and only the significant differences are discussed below.

Referring now to FIGS. 12 and 14 together, in accordance with this embodiment, valve prosthesis 1400 is essentially identical to valve prosthesis 1200 except that valve prosthesis 1400 does not include trident posts 354, protruding commissures 1260, and trident stitching 1262. In accordance with this embodiment, commissures 360 are attached to axial frame members 352 of commissure posts 340 with commissure stitching 370. Commissures 360 are attached to axial frame members 352 inferior to inferior crowns 350 where outflow crown struts 346 connect to commissure posts 340.

Similar to valve prosthesis 1200, in valve prosthesis 1400, free edges 362 of valve leaflets 310 are located inferior from outflow crown ring 338 such that thus there is no overlap of or contact between valve leaflets 310 and outflow crown ring 338.

By avoiding overlap of and contact between valve leaflets 310 and outflow crown ring 338, as outflow crown ring 338 opens during deployment, damage, e.g., shearing, of free edges 362 of valve leaflets 310 from outflow crown ring 338 is avoided. Further, pinning of valve leaflets 310 between the expanding balloon and outflow crown ring 338 during deployment is avoided. Pinning can prevent valve leaflets 310 from unfolding and causes stresses at free edges 362 and leaflet commissures 360 which causes "peeling", "splits", "cuts", "sheering", "tensile failure" and/or other damage to valve leaflets 310.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Further disclosed herein is the subject-matter of the following clauses:
1. A valve prosthesis comprising:
   a one piece molded prosthetic valve; and
   a stent structure comprising an outflow portion, the outflow portion being tapered and having a minimum diameter at an outflow end of the valve prosthesis.
2. The valve prosthesis of Clause 1 wherein the stent structure further comprises an inflow portion, the outflow portion having a first diameter where the outflow portion connects to the inflow portion and a second diameter at the outflow end, the second diameter being smaller than the first diameter.
3. The valve prosthesis of Clause 2 wherein the outflow portion uniformly tapers between the first diameter and the second diameter.
4. The valve prosthesis of Clause 2 wherein the inflow portion has a uniform diameter.
5. The valve prosthesis of Clause 1 or of any of the preceding Clauses wherein the stent structure further comprises struts of different lengths that upon expansion cause the outflow portion to be tapered.
6. The valve prosthesis of Clause 1 or of any of the preceding Clauses wherein the valve prosthesis has a first diameter at an inflow end of the valve prosthesis and a second diameter at the outflow end of the valve prosthesis, the valve prosthesis uniformly tapering between the first diameter and the second diameter.
7. A method comprising:
   expanding a balloon to deploy a valve prosthesis comprising a one piece molded prosthetic valve, wherein upon deployment, an outflow portion of a stent structure of the valve prosthesis is tapered and has a minimum diameter at an outflow end of the valve prosthesis, the valve prosthesis assuming a shape of the balloon.
8. The method of Clause 7 wherein the balloon has a tapered outer surface having a diameter at a distal end greater than a diameter at a proximal end when inflated.
9. The method of Clause 8 wherein the valve prosthesis has a first diameter at an inflow end of the valve prosthesis and second diameter at the outflow end of the valve prosthesis, the valve prosthesis uniformly tapering between the first diameter and the second diameter.
10. The method of Clause 7 wherein the balloon is partially tapered when inflated.
11. The method of Clause 10 wherein the balloon has a cylindrical distal portion and a proximal tapered portion.
12. The method of Clause 11 wherein the stent structure further comprises an inflow portion having a uniform diameter.
13. A valve prosthesis comprising:
   a one piece molded prosthetic valve; and
   a stent structure comprising an outflow crown ring, the outflow crown ring comprising:
      inferior crowns;
      superior crowns; and
      outflow crown struts connected to the superior crowns and the inferior crowns, an internal radius of the superior crowns being within a range of 0.3 to 0.5 millimeters (mm).
14. The valve prosthesis of Clause 13 wherein the superior crowns lie within a cylindrical shape of the stent structure.
15. The valve prosthesis of Clause 14 wherein the superior crowns are atraumatic.
16. A valve prosthesis comprising:
   a one piece molded prosthetic valve comprising:
      valve leaflets comprising free edges; and
   a stent structure comprising an outflow portion comprising an outflow crown ring, the free edges being inferior to the outflow crown ring.
17. The valve prosthesis of Clause 16 wherein there is a longitudinal space between the valve leaflets and the outflow crown ring.
18. The valve prosthesis of Clause 16 or of any of Clauses 16-17 wherein the prosthetic valve further comprises protruding commissures attached to trident posts of the outflow portion of the stent structure.
19. The valve prosthesis of Clause 18 wherein the prosthetic valve further comprises commissures attached to axial frame members of the outflow portion.
20. The valve prosthesis of Clause 16 or of any of Clauses 16-19 wherein the prosthetic valve further comprises commissures attached to commissure posts inferior to the outflow crown ring.

## Claims

1. A valve prosthesis comprising:
a one piece molded prosthetic valve; and
a stent structure comprising an outflow portion, the outflow portion being tapered and having a minimum diameter at an outflow end of the valve prosthesis.

2. The valve prosthesis of Claim 1, wherein the stent structure further comprises an inflow portion, the outflow portion having a first diameter where the outflow portion connects to the inflow portion and a second diameter at the outflow end, the second diameter being smaller than the first diameter.

3. The valve prosthesis of Claim 2, wherein the outflow portion uniformly tapers between the first diameter and the second diameter.

4. The valve prosthesis of Claim 2 wherein the inflow portion has a uniform diameter.

5. The valve prosthesis of any of the preceding Claims, wherein the stent structure further comprises struts of different lengths that upon expansion cause the outflow portion to be tapered.

6. The valve prosthesis of any of the preceding Claims, wherein the valve prosthesis has a first diameter at an inflow end of the valve prosthesis and a second diameter at the outflow end of the valve prosthesis, the valve prosthesis uniformly tapering between the first diameter and the second diameter.

7. A valve prosthesis comprising:
a one piece molded prosthetic valve; and
a stent structure comprising an outflow crown ring, the outflow crown ring comprising:
inferior crowns;
superior crowns; and
outflow crown struts connected to the superior crowns and the inferior crowns, an internal radius of the superior crowns being within a range of 0.3 to 0.5 millimeters (mm).

8. The valve prosthesis of Claim 7 wherein the superior crowns lie within a cylindrical shape of the stent structure.

9. The valve prosthesis of Claim 8 wherein the superior crowns are atraumatic.

10. A valve prosthesis comprising:
a one piece molded prosthetic valve comprising:
valve leaflets comprising free edges; and
a stent structure comprising an outflow portion comprising an outflow crown ring, the free edges being inferior to the outflow crown ring.

11. The valve prosthesis of Claim 10 wherein there is a longitudinal space between the valve leaflets and the outflow crown ring.

12. The valve prosthesis of any of Claims 10-11 wherein the prosthetic valve further comprises protruding commissures attached to trident posts of the outflow portion of the stent structure.

13. The valve prosthesis of Claim 12 wherein the prosthetic valve further comprises commissures attached to axial frame members of the outflow portion.

14. The valve prosthesis of any of Claims 10-13 wherein the prosthetic valve further comprises commissures attached to commissure posts inferior to the outflow crown ring.
